# EUROPEAN PATENT SPECIFICATION

(11) **EP 2 010 136 B1**
(45) Date of publication and mention of the grant of the patent: **12.09.2012**
(21) Application number: 07755124.0
(22) Date of filing: 10.04.2007
(51) Int. Cl.: A61K 8/49, A61K 31/517, A61Q 19/06, A61K 36/00, A61P 17/00

(54) **METHODS FOR MODULATING FORMATION AND PROGRESSION OF CELLULITE**
VERFAHREN ZUR MODULIERUNG DER ENTSTEHUNG UND PROGRESSION VON CELLULITE
PROCÉDÉS DE MODULATION DE LA FORMATION ET DE LA PROGRESSION DE LA CELLULITE

(30) Priority: 10.04.2006 US 791096 P
(43) Date of publication of application: 07.01.2009
(73) Proprietor: The President and Fellows of Harvard College, Cambridge, MA 02138 (US)
(72) Inventor: WHITMAN, Malcolm, Jamaica Plain, MA 02130 (US); KELLER, Tracy, Jamaica Plain, MA 02130 (US)
(74) Representative: Bevan, Emma
(86) International application number: PCT/US2007/008752
(87) International publication number: WO 2007/120606

(56) References cited:
- WO-A-00/09070
- WO-A1-01/17498
- WO-A2-03/016860
- US-A- 5 449 678
- US-B1- 6 358 539
- PINES M ET AL: "Reduction in dermal fibrosis in the tight-skin (Tsk) mouse after local application of halofuginone." BIOCHEMICAL PHARMACOLOGY 1 NOV 2001, vol. 62, no. 9, 1 November 2001 (2001-11-01), pages 1221-1227, XP002451804 ISSN: 0006-2952
- CÉLINE VIENNET ET AL: "Contractile forces generated by striae distensae fibroblasts embedded in collagen lattices", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 297, no. 1, 1 July 2005 (2005-07-01), pages 10-17, XP019341129, ISSN: 1432-069X, DOI: 10.1007/S00403-005-0557-9
- MARK EASTWOOD ET AL: 'Quantitative analysis of collagen gel contractile forces generated by dermal fibroblasts and the relationship to cell morphology' JOURNAL OF CELLULAR PHYSIOLOGY vol. 166, no. 1, 01 January 1996, pages 33 - 42, XP055015938 ISSN: 0021-9541
- CAMPBELL BRIAN H ET AL: "A multi-station culture force monitor system to study cellular contractility.", JOURNAL OF BIOMECHANICS JAN 2003 LNKD- PUBMED:12485649, vol. 36, no. 1, January 2003 (2003-01), pages 137-140, ISSN: 0021-9290
- MARCUS JARMAN-SMITH ET AL: 'Human fibroblast culture on a crosslinked dermal porcine collagen matrix' BIOCHEMICAL ENGINEERING JOURNAL vol. 20, no. 2-3, 01 August 2004, pages 217 - 222, XP055015952 ISSN: 1369-703X

## Description

### Field of the Invention

This invention relates generally to methods for modulating the formation and/or progression of cellulite and stretch marks in a subject.

### Background of the Invention

Cellulite is a skin condition that is characterized by dimpling and puckering of the skin of the thighs and buttocks. Cellulite is rare in men and quite common in women. Thus, cellulite poses a great cosmetic concern to a large number of people due to its prevalence.

Purported treatments for cellulite are currently being marketed, but these current treatments are often very expensive. Treatments include, e.g., topical ointments such as aminophylline cream, which attempt to alter adipose tissue metabolism. Other treatment methods include physical and mechanical treatment methods, surgery, and laser treatment of an area exhibiting cellulite. However, no effective strategy has been described to date for the prevention or long-term management of cellulite.

Accordingly, significant need exists for methods for modulating the formation and/or presence of cellulite and other skin disorders in a subject.
WO 00/09070 A describes quinazoline derivatives to treat pathogenic aspects of tissue trauma such as fibrotic conditions, formulations of adhesions, keloids, hypertrophic scars and collagen deposition. Pines M et al., Biochemical Pharmacology, vol.62, no.9, 2001, p.1221-1227 describes the dermal application of halofuginone for use in the reduction of collagen alpha (I) gene expression and the proliferation of dermal fibroblasts.
US-A-5,449,678 describes compositions containing quinazolinones, effective to inhibit collagen type II synthesis, which may be used to treat diseases caused by excessive collagen type I deposits or fibrosis.

### Summary of the Invention

The present invention provides methods employing compounds of formula (I) for decreasing the appearance of cellulite and preventing or decreasing the appearance of stretch marks in a subject. The term "skin disorders" refers to a disorder that manifests itself as an aberrant or irregular appearance of the skin of an individual, but the disorder itself poses little or no health risk in and of itself and doesn't compromise or otherwise impede the body's ability to function normally and repair itself. A skin disorder to be treated or otherwise modulated by the methods described herein include disorders that affect the appearance and/or structure of any layer of the skin, e.g., the epidermis, the dermis, the hypodermis, any of the sublayers of epidermis such as the stratum basale, the stratum spinosum, the stratum granulosum, the stratum licidum, and the stratum corneum, and any combination thereof.
Skin disorders that are modulated, *e.g*., reduced, inhibited, treated or prevented, by the methods provided herein are cellulite and stretch marks (*striae distensae*).

The invention features a method for decreasing the appearance of cellulite in a subject exhibiting cellulite, by identifying a tissue treatment site in the patient, wherein the tissue treatment site includes at least one area in which the appearance of cellulite occurs, contacting the tissue treatment site with a compound of formula (I), where the treated tissue site exhibits a decreased contractile force exerted by vertical collagen fibers tethered between fascia and dermo-bypodermal interface within the tissue treatment site, as compared to a contractile force exerted by vertical collagen fibers in a second tissue site exhibiting cellulite in the subject. In various aspects, the method also includes exposing the tissue treatment site to a laser, and/or exposing the tissue treatment site to mechanical manipulation.

The invention provides methods for treating or preventing cellulite in a subject in need thereof by administering to the subject a compound of formula (I) in an amount effective to modulate binding of halofuginone and the target.

The compound used in tissue treatment or other treatment and/or prevention of a cellulite or other skin disorders is a compound according to formula I: 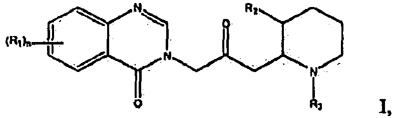 or a salt, isomer, or solvate thereof, where R₁ is hydrogen, halogen, nitro, benzo, lower alkyl, phenyl or lower alkoxy; R₂ is hydroxy, acetoxy, or lower alkoxy, R₃ is hydrogen, and n is 1, 2, 3 or 4. The compound is administered in an amount effective to modulate the formation and/or progression of cellulite in a subject. For example, the compound is febrifugine or halofuginone.

In various aspects, the compound used for decreasing the appearance of cellulite in a subject inhibits maturation of myofibroblasts. Such compounds are referred to herein as "myofibroblast maturation inhibitors" or "myofibroblast maturation inhibitory compounds". For example, the myofibroblast maturation inhibitory compound used for decreasing the appearance of cellulite in a subject compound inhibits one or more biological activities of myofibroblasts, such as expression of actin-containing stress fibers, expression and organization of fibronectin into fibril, and formation of large fibronexus adhesion complexes.

In various aspects, the compound used for decreasing the appearance of cellulite in a subject modulates extracellular matrix (ECM) remodeling. These compounds are referred to herein as "ECM remodeling compounds". It should be noted that the terms "myofibroblast maturation inhibitory compound" and "ECM remodeling compound" are not mutually exclusive - a compound of the invention that modulates the formation and/or progression of cellulite in a subject can be both a myofibroblast maturation inhibitory compound and an ECM remodeling compound.

The myofibroblast maturation inhibitory compounds, ECM remodeling compounds and myofibroblast maturation inbibitory/ECM remodeling compounds of the invention include compounds of Formula I, and in particular, compounds that are structurally related to and/or derived from halofuginone.

The compound used in the methods of the invention is formulated for topical administration, for example, as a film, membrane, foam, gel, or cream.

### Brief Description of the Figures

Figure 1 is an illustration depicting the chemical structure of halofuginone (HF). Potential sites for chemical derivatization are indicated by the numbers 1-4.

Figures 2A and 2B are a series of illustrations depicting the inhibition of the adhesive and/or stretching properties of primary dermal fibroblasts by halofuginone.

Figure 3 is an illustration depicting the purification of TIF1β (TIF1-beta), a binding protein for halofuginone.

Figure 4 is an illustration depicting the amino acid sequence of the HF binding protein, TIF1 β.

Figures 5A and 5B are a series of illustrations depicting validation of TIF1 β as a binding protein that is specific for halofuginone. Figure 5A is an illustration depicting the clone, tagged TIF1 β that was transfected into SV-MES cells. Figure 5 is an illustration depicting the interaction between HF and TIF1 β.

Figure 6 is an illustration depicting the ability of HF to inhibit myofibroblast formation in response to TGFß.

Figure 7 is a series of illustrations depicting the behavior of TGFB treated dermal fibroblasts in a collagen matrix in the presence (right panel) or absence (left panel) of HF.

Figure 8 is a graph depicting the ability of HF to inhibit contractile activity of myofibroblasts in a collagen matrix.

### Detailed Description of the Invention

The present invention provides methods for modulating, *e.g*., reducing, inhibiting, treating or preventing, the formation and/or progression of cellulite and stretch marks in a subject.

### Cellulite

Cellulite is a skin disorder that is characterized by dimpling and puckering of the skin of the thighs and buttocks. It is rare in men and quite common in women, with the estimated frequency of 85-98% of post-pubertal females of all races being affected to some extent (Avram, M, J. Cosmet Laser Ther, vol. 6: 181-185 (2004)). Cellulite is a poorly understood and controversial topic; purported treatments abound, but are expensive. While different hypotheses exist to explain its etiology, there is no consensus opinion regarding the cause or progression of cellulite (*See e.g.,* Avram, M, J. Cosmet Laser Ther, vol. 6: 181-185 (2004); Querleux, et al. Skin Res Technol 8, 118-24 (2002); Mirrashed, et al. Skin Res Technol 10, 161-8 (2004); and Smalls, J Cosmet Sci 56, 105-20 (2005)). Increased percentages of thigh fat are associated with a worsening of this condition. Cellulite is not simply a function of adipose volume, however, because very obese men rarely demonstrate cellulite and extremely slender women with good muscle tone can evidence marked dimpling (Rosenbaum, et al. Surg, vol. 101: 1934-39 (1998)). Even though cellulite is not a pathology, it poses a great cosmetic concern to a large number of people due to its prevalence. No effective strategy has been described to date for the prevention or long-term management of this condition.

Cellulite may be the result of gender differences in the biochemistry of either adipose tissue (fat) or connective tissue. While many popular treatments target the adipose component of this condition, there is no indication that adipose tissue in affected areas or individuals has an increased water content, or is in any way physiologically different from unaffected adipose tissue (Rosenbaum, et al. Surg, vol. 101: 1934-39 (1998); and Querleux, et al. Skin Res Technol 8, 118-24 (2002)). Microanatomical descriptions of cellulite vary significantly in the literature and often conflict with one another (Smalls, J Cosmet Sci 56, 105-20 (2005); Pierard, et al. Am J Dermatopathol 22, 34-7 (2000)); Rosenbaum, et al. Surg, vol. 101: 1934-39 (1998); Querleux, et al. Skin Res Technol 8, 118-24 (2002); and Mirrashed, et al. Skin Res Technol 10, 161-8 (2004)). Studies include histologic examination of samples (from autopsy specimens and/or live biopsy material, containing small numbers of dubiously matched controls), high-resolution magnetic resonance imaging (MRI), and sonography of skin regions affected by cellulite (Pierard, et al. Am J Dermatopathol 22, 34-7 (2000); Rosenbaum, et al. Surg, vol. 101: 1934-39 (1998); Querleux, et al. Skin Res Technol 8, 118-24 (2002); and Mirrashed, et al. Skin Res Technol 10, 161-8 (2004)).

Studies have demonstrated the existence of microanantomical differences between men and women at the connective tissue border that separates the dermis from the subcutaneous fat-containing hypodermis of thighs and buttocks (Pierard, et al. Am J Dermatopathol 22, 34-7 (2000); Rosenbaum, et al. Surg, vol. 101: 1934-39 (1998)). These differences underlie the sexual dimorphism seen in the presentation of the cellulite condition. While men (and a small percentage of women) have a smooth, continuous dermo-hypodermal interface and are unaffected by cellulite, the majority of women have discontinuities in this region that are accompanied by varying degrees of protrusion of adipose tissue into the dermis. Increased protrusion of fat into the dermis is eventually associated with the appearance and worsening of the condition cellulite. In women, and in the rarely affected men, this structural discontinuity is thought to develop at puberty and to be determined hormonally (Numberger, et al. J Dermatol Surg Oncol 4, 221-9 (1978); Rosenbaum, et al. Surg, vol. 101: 1934-39 (1998)). Regardless of whether female subjects currently are affected by cellulite, the vast majority of them possess these gender-linked discontinuities in the dermo-hypodermal interface, along with mild protrusion of fat into the dermis (Pierard, et al. Am J Dermatopathol 22, 34-7 (2000)). Furthermore, female subjects who are affected by cellulite demonstrate this pattern of connective tissue irregularity diffusely, both in affected and unaffected areas of the thigh (Rosenbaum, et al. Surg, vol. 101: 1934-39 (1998)). These observations indicate that, while the presence of connective tissue discontinuity at the dermo-hypodermal border is coincident with susceptibility to cellulite, this structural feature alone is insufficient to produce the skin dimpling that is the central defining characteristic of cellulite.

Structural differences between men and women in the connective tissues that tether the dermis to the superficial fascia(Mirrashed, et al. Skin Res Technol 10, 161-8 (2004); Pierard, et al. Am J Dermatopathol 22, 34-7 (2000); Querleux, et al. Skin Res Technol 8, 118-24 (2002); Rosenbaum, et al. Surg, vol. 101: 1934-39 (1998)). Connective tissue structural disparities between skin samples that either are, or are not, affected by cellulite also have been observed within the hypodermal space.

### Model for Cellulite Formation and Progression

Gender differences in mechanical regulation of tensional homeostasis and dynamic tissue remodeling in the hypodermis result in skin dimpling. The hypodermis is the layer of subcutaneous fat that lies under the dermis, but above the fascia that surrounds muscle, and the periosteum that surrounds bone (Kanitakis, et al. Eur J Dermatol 12, 390-9; quiz 400-1 (2002)). In addition to fat lobules, the hypodermal space is composed of a vertically oriented 3- dimensional lattice network of fibrous elements connecting the reticular dermis with the superficial fascia. The hypodermal vertical collagen network is arranged in geometric subunits, allowing for skin mobility, and serving as an expansive-retractile reservoir for the deposition and absorption of fat. Connective tissue structures normally observed in this space include septae, composed of thick parallel collagen bundles, and smaller microfibrils, composed of collagen and elastin. Connective tissues are under mechanical tension even at rest, as evidenced by the fact that they do not, for the most part, sag. Maintenance of this mechanical load is called tensional homeostasis, and it must be reestablished within the extracellular matrix after tissue injury, during repair. While tissues and ECM are stress-loaded, the resident cells of mature connective tissue are largely stress-shielded by the matrix that they deposit and remodel (Tomasek, et al., Nat Rev Mol Cell Biol 3, 349-63 (2002)). Fibroblasts that migrate during growth or in response to injury, however, become subject to mechanical forces, which can induce them to differentiate into myofibroblasts that possess contractile properties (Tomasek, et al., Nat Rev Mol Cell Biol 3, 349-63 (2002)). The myofibroblast phenotype is regulated by mechanical tension *in vivo,* as well as by the local release of some cytokines. Myofibroblasts are capable of reorganizing collagen networks along planes of mechanical tension, and can exert contractile force (Desmouliere, et al., Wound Repair Regen., vol. 13: 7-12 (2005); Hinz, et al. Thromb Haemost, vol 90: 993-1002 (2003); Hinz, et al., Curr Opin Biotechnol., vol. 14: 538-46 (2003); Tomasek, et al., Nat Rev Mol Cell Biol 3, 349-63 (2002)). Existing collagen fibers within the ECM also will align themselves along newly generated stress planes.

The model for cellulite formation, progression, and treatment described is consistent with observations reported in the literature and the data presented herein. While not intending to be bound by theory, it is believed that a majority of post-pubertal females of a given body mass index have a discontinuous dermo-hypodermal interface accompanied by regions of modest adipose protrusion. This discontinuous connective tissue border predisposes women to the eventual development of cellulite by destabilizing tensional homeostasis in the adipose compartment, or hypodermal space, of the thigh and buttock. While there are certainly structural differences between women, the predominant factor in the development of cellulite is a variable degree of response to the trigger of fat protrusion at the dermal adipose junction. In a women's thigh, the fragile, structurally compromised connective tissue border micro-anatomically "gives way" in response to a stimulus, such as the addition of fat to the area or regional mechanical stress, which can elicit varying degrees of response from a given tissue, in a given woman.

What follows is a dynamic process resulting in the formation and progression of cellulite, a process that is modulated, *e.g*., interrupted or reversed, pharmacologically. Local cytokine release occurs at the site of fat protrusion and connective tissue destruction, and these local cytokines initiate fibroblast migration in the vertical plane. Either cytokine release, or exposure to the mechanical forces of migration, triggers the differentiation/maturation of fibroblasts to myofibroblasts, as well as stimulates the increased deposition of ECM. The directed movement of fibroblasts generates a tractional force, thereby creating stress planes that initiate vertical realignment of existing hypodermal lattice structure-*i.e*., collagen microfibrils, and septae. The newly differentiated myofibroblasts express actin-containing stress fibers, express and organize fibronectin into fibrils, and form large fibronexus adhesion complexes that allow the generation of contractile force. Contractile myofibroblasts restore tensional homeostasis to the hypodermis through mechanical re-loading of the ECM. The net result of this process is to tether the dermis more tightly to the underlying fascia, creating skin dimples that are the characteristic sign of cellulite. Contractile force generation by collagen bundles tethered between the fascia and the dermo-hypodermal interface also leads to further protrusion of subcutaneous fat, and further destruction of the connective tissue border, creating a biomechanical feedback loop of fat protrusion, dimpling, and loss of elasticity within the hypodermal vertical lattice. Depending on the extent of response, myofibroblasts persist, and vertical collagen fibers thicken, becoming increasingly fibrotic. Microanatomical observations of cellulite-affected tissue (Pierard, et al. Am J Dermatopathol 22, 34-7 (2000); Querleux, et al. Skin Res Technol 8, 118-24 (2002); Rosenbaum, et al. Surg, vol. 101: 1934-39 (1998)) reflect this mechanism. In the extreme presentation, normal hypodermal connective tissue structures are remodeled entirely, and replaced with a tortuous, fibrotic network of collagen fibers. Consistent with all observations, this process is greatly exacerbated by increased fat deposition and losses of elasticity in the affected tissues.

A common perception in the art is that collagen-promoting factors are favorable to improving the appearance of cellulite, and that the connective tissue structures involved in cellulite formation are intractable. However, in one aspect of the current invention, dynamic tightening, and loss of elasticity in the connective tissue in the hypodermis is responsible for driving the protrusion of fat through the dermal-hypodermal interface. This process is treated using an ECM-remodeling compound, such as those described herein.

In the methods described herein, cellulite is treated with pharmacologic intervention, using small molecules that modify the behavior of fibroblasts and myofibroblasts in the dermis and hypodermis. The treatment model described herein is the only method, to date, that targets the formation of skin dimples in the thigh and buttock, by targeting the structures that define the condition cellulite. By using small molecule inhibitors to interfere with either the maturation or the action of myofibroblasts, dynamic processes that form the basis for cellulite appearance and progression- tissue remodeling of the hypodermal ECM, and progressive contractile force generation upon the dermo-hypodermal connective tissue interface - are modulated, *e.g*., prevented or reversed.

### Stretch Marks

Stretch marks, also known as *striae distensae,* are a common disfiguring condition associated with continuous and progressive stretching of the skin. Stretch marks are commonly due to stretching of the skin, as in rapid weight gain, or mechanical stress, as in weight lifting.

Stretch marks are skin defects that look like bands, stripes, or lines. Stretch marks are seen when a person grows or gains weight rapidly or has certain diseases or conditions. Common causes for stretch marks include pregnancy, puberty, obesity, Cushing's syndrome (an endocrine disorder caused by high levels of cortisol in the blood), Iatrogenic Cushing syndrome, overuse of topical corticosteroid, and Ehlers-Danlos syndrome (a group of rare genetic syndromes caused by a defect in collagen synthesis).

Men and women can get stretch marks on several areas of their bodies, including the abdominal area, thighs, hips, breasts, upper arms or lower back.

Purported treatment of stretch marks are also currently being marketed, but these treatment are available solely for the purpose of improving the appearance of existing stretch marks. Such treatments for improving the appearance of existing stretch marks include laser treatments, dermabrasion, and topical applications of compositions such as cocoa butter. However, no effective strategy has been described to date for the prevention or cure of stretch marks.

In the methods described herein, stretch marks are treated, *e.g*., the appearance and fibrosity of the stretch mark or marks is reduced, using pharmacologic intervention using small molecules that modify the behavior of fibroblasts and myofibroblasts in the reticular dermis. Studies have attempted to identify the distribution of extracellular matrix components in skin affected by stretch marks (Watson, et al., Br. J. Dermatol., vol. 138(6):931-937 (1998)), and the contractile forces properties of fibroblasts from stretch marks (Viennet, et al., Arch. Dermatol. Res., vol-297(1): 10-17 (2005)). However, the treatment model described herein is the only method, to date, that targets the formation of striae in a subject by targeting the structures that define the condition. By using small molecule inhibitors to interfere with either the maturation or the action of myofibroblasts, dynamic processes that form the basis for striae appearance and progression are modulated, e.g., prevented or reversed.

### Model for Stretch Mark Formation and Progression

In the model for stretch mark formation provided herein, stretch marks are caused by a process of extracellular fiber breakage and tissue micro-tears that, unlike cellulite, occurs primarily in the horizontal plane of the reticular dermis concomitant with skin expansion. As with the proposed model for cellulite, however, a buttressing reaction of myofibroblast-driven contractile ECM deposition is central to the presentation of the condition that manifests as stretch marks. HF, chemically-modified HF of formula (I) and febrifugine that act by diminishing the presence or function of contractile myofibroblasts in the reticular dermis prevent, inhibit progression of, and/or improve the appearance of stretch marks.

### Methods for modulating the formation and/or progression of cellulite and other skin disorders

Suitable modulators of the formation and/or progression of cellulite and other skin disorders include, for example, compositions containing quinazolinones. We therefore disclose an anti-cellulite and/or anti-striae composition comprising an amount of quinazolinone derivative as herein defined, effective to inhibit cellulite formation and/or progression, which is therefore useful as a cosmetic composition.

The invention includes a method for reducing the appearance of or removing cellulite, by administering an effective amount of an anti-cellulite composition comprising a compound of formula I: 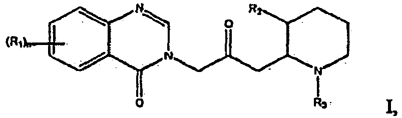 wherein: R₁ is selected from hydrogen, halogen, nitro, benzo, lower alkyl, phenyl and lower alkoxy;
R₂ is selected from hydroxy, acetoxy, and lower alkoxy,
R₃ is hydrogen, and
n is selected from 1, 2, 3 and 4;
in an amount effective to modulate the formation and/or progression of cellulite in a subject.

The invention includes a method for preventing the appearance of or for reducing the appearance of stretch marks, by administering an effective amount of an anti-striae composition comprising a compound of formula I: 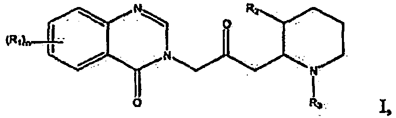 wherein: R₁ is selected from hydrogen, halogen, nitro, benzo, lower alkyl, phenyl and lower alkoxy;
R₂ is selected from hydroxy, acetoxy, and lower alkoxy,
R₃ is hydrogen, and
n is selected from 1, 2, 3 and 4;
in an amount effective to modulate the formation and/or progression of cellulite in a subject.

The compositions used in the methods of the invention include compounds of formula I and salts, isomers, and solvates thereof.

The compositions used in the methods of the invention include acid addition salts.

In certain compounds, n is one. In other compounds, n is two.

In various compounds according to formula I, R₁ is halogen. For example, n is two and both substituents are halogen.

Certain compositions useful in the methods of the invention include an acid addition salt of a compound of formula I. For example, the acid addition salt is a hydrobromide salt.

For example, a compound according to formula I is halofuginone:

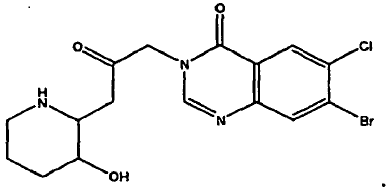

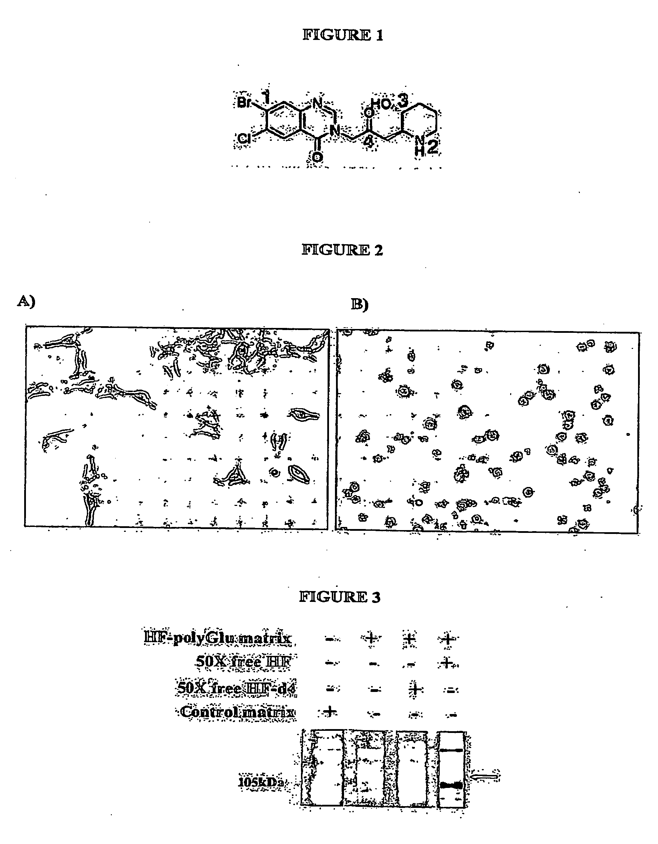

*Halofuginone.* Halofuginone (HF) (Fig 1.) is a halogenated derivative of febrifugine, a natural product extracted from the roots of the hydrangea Dichroa febrifuga. Dichroa febrifuga is one of the "fifty fundamental herbs" of traditional Chinese medicine, originally used as an anti-malarial remedy (Jiang, et al. Antimicrob Agents Chemother 49, 1169-76 (2005)). Halofuginone, otherwise known as 7-bromo-6-chloro-3-[3-(3-hydroxy-2-piperidinyl)-2-oxopropyl]-4(3H)-quinazolinone, and halofuginone derivatives were described and claimed in U.S. Pat. No. 3,320,124. Febrifugine has been shown to be the active ingredient in Dichroa febrifuga extracts; HF was originally synthesized in search of less toxic anti-malarial derivatives of febrifugine. In addition to its anti-malarial properties, however, HF has striking anti-fibrotic properties *in vivo.* HF potently reduces dermal extracellular matrix (ECM) deposition with low *in vivo* toxicity, which has led to investigation of its utility as a therapeutic for fibrosis, the pathological deposition of ECM (Pines, et al. Biol Blood Marrow Transplant 9, 417-25 (2003)). HF inhibits the transcription of a number of components and modulators of ECM function, including Type I collagen, fibronectin, the matrix metallopeptidases MMP-2 and MMP-9, and the metalloprotease inhibitor TIMP-2 (Li, et al. World J Gastroenterol 11,3046-50 (2005); Pines, et al. Biol Blood Marrow Transplant 9, 417-25 (2003)). The major cell types responsible for altered ECM deposition, tissue thickening, and contraction during fibrosis are fibroblasts and myofibroblasts. Myofibroblasts mature/differentiate from their precursor fibroblasts in response to cytokine release, often following tissue damage, and mechanical stress, and can be distinguished from fibroblasts by their contractile activity. Excess deposition of ECM, and the differentiation of myofibroblasts that possess contractile activity are central features of fibrosis in a wide range of organs and pathological conditions (Border, et al., New England J. Med., vol. 331: 1286-92 (1994); Branton, et al., Microbes Infect., vol. 1: 1349-65 (1999); Flanders, Int J Exp Pathol vol. 85: 47-64 (2004)). HF, therefore, has been studied extensively as a potential anti-fibrotic therapeutic, and has progressed to phase 2 clinical trials for applications stemming from these properties.

HF acts potently as an inhibitor of fibrosis, at concentrations in the range of 20-200 nM *in vitro,* and acts specifically, demonstrating low-toxicity *in vivo* (Pines, et al. Biol Blood Marrow Transplant 9, 417-25 (2003)). In animal models of wound healing and fibrotic disease, HF reduces excess dermal ECM deposition when introduced intraperitoneally, added to food, or applied locally (Pines, et al. Biol Blood Marrow Transplant 9, 417-25 (2003)). The low toxicity of HF suggests that it does not block any general cellular functions at the doses used for inhibition of fibrosis. HF is currently in Phase II clinical trials as a treatment for scleroderma (Pines, et al. Biol Blood Marrow Transplant 9, 417-25 (2003)), bladder cancer (Elkin, et al., Cancer Res., vol. 59: 4111-18 (1999)), and angiogenesis during Kaposi's Sarcoma, as well as in earlier stages of clinical investigation for a wide range of fibrosis-associated disorders (Nagler, et al. Am J Respir Crit Care Med 154, 1082-86 (1996); Nagler, et al. Arterioscler Thromb Vasc Biol 17, 194-202 (1997); Nagler, et al. Eur J Cancer 40, 1397-403 (2004); Ozcelik, et al. Am J Surg 187, 257-60 (2004)). In spite of the excellent therapeutic promise of HF, very little is known about the molecular mechanisms of HF action. An important recent development has been the demonstration that HF can antagonize the pro-fibrotic activity of the cytokine TGFß (Xavier, et al., J Biol Chem 279, 15167-76 (2004)) (McGaha, et al. J Invest Dermatol 118, 461-70 (2002)).

As used herein, the term "alkyl" includes saturated aliphatic groups, including straight-chain alkyl groups (*e.g*., methyl, ethyl, propyl, butyl, pentyl, hexyl) and branched-chain alkyl groups (*e.g*., isopropyl, tert-butyl, isobutyl. In certain embodiments, a straight chain or branched chain alkyl has six or fewer carbon atoms in its backbone (*e.g*., C₁-C₆ for straight chain, C₃-C₆ for branched chain), and in other embodiments four or fewer carbon atoms. Lower alkyl groups include from 1-6 carbon atoms, thus the term "lower alkyl" includes alkyl groups containing 1, 2, 3, 4, 5, or 6 carbon atoms.

The term "alkoxy" or "alkoxyl" includes substituted and unsubstituted alkyl groups covalently linked to an oxygen atom. Examples of alkoxy groups (or alkoxyl radicals) include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, carboxylate, alkoxyl, cyano, amino (including -NH₂, alkylamino, dialkylamino, arylamino, diarylamino, and alkylarylamino), nitro, trifluoromethyl, cyano, azido, heterocyclyl, or an aromatic or heteroaromatic moiety. Examples of halogen substituted alkoxy groups include, but are not limited to, fluoromethoxy, difluoromethoxy, trifluoromethoxy, chloromethoxy, dichloromethoxy, and trichloromethoxy. Lower alkoxy groups include from 1-6 carbon atoms, thus the term "lower alkoxy" includes alkyl groups containing 1, 2, 3, 4, 5, or 6 carbon atoms.

The term "hydroxy" or "hydroxyl" includes groups with an -OH or -O⁻.

The term "halogen" includes fluorine, bromine, chlorine, iodine, etc. The term "perhalogenated" generally refers to a moiety wherein all hydrogens are replaced by halogen atoms.

In the present specification, the structural formula of the compound represents a certain isomer for convenience in some cases, but the present invention includes all isomers such as geometrical isomer, optical isomer based on an asymmetrical carbon, stereoisomer, tautomer and the like which occur structurally and an isomer mixture and is not limited to the description of the formula for convenience, and may be any one of isomer or a mixture. Therefore, an asymmetrical carbon atom may be present in the molecule and an optically active compound and a racemic compound may be present in the present compound, but the present invention is not limited to them and includes any one. In addition, a crystal polymorphism may be present but is not limiting, but any crystal form may be single or a crystal form mixture, or an anhydride or hydrate.

It will be noted that the structure of some of the compounds of the invention include asymmetric (chiral) carbon atoms. It is to be understood accordingly that the isomers arising from such asymmetry are included within the scope of the invention, unless indicated otherwise. Such isomers can be obtained in substantially pure form by classical separation techniques and by stereochemically controlled synthesis. The compounds of this invention may exist in stereoisomeric form, therefore can be produced as individual stereoisomers or as mixtures.

"Isomerism" means compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

"Chiral isomer" means a compound with at least one chiral center. It has two enantiomeric forms of opposite chirality and may exist either as an individual enantiomer or as a mixture of enantiomers. A mixture containing equal amounts of individual enantiomeric forms of opposite chirality is termed a "racemic mixture". A compound that has more than one chiral center has 2ⁿ⁻¹enantiomeric pairs, where n is the number of chiral centers. Compounds with more than one chiral center may exist as either an individual diastereomer or as a mixture of diastereomers, termed a "diastereomeric mixture". When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al, Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J., Chem. Educ. 1964, 41, 116).

"Geometric Isomers" means the diastereomers that owe their existence to hindered rotation about double bonds. These configurations are differentiated in their names by the prefixes cis and trans, or Z and E, which indicate that the groups are on the same or opposite side of the double bond in the molecule according to the Cahn-Ingold-Prelog rules.

Further, the structures and other compounds discussed in this application include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixtures, however as a result of recent advances in chromatography techniques, it has been possible to separate mixtures of two atropic isomers in select cases.

The terms "crystal polymorphs" or "polymorphs" or "crystal forms" means crystal structures in which a compound (or salt or solvate thereof) can crystallize in different crystal packing arrangements, all of which have the same elemental composition. Different crystal forms usually have different X-ray diffraction patterns, infrared spectral, melting points, density hardness, crystal shape, optical and electrical properties, stability and solubility. Recrystallization solvent, rate of crystallization, storage temperature, and other factors may cause one crystal form to dominate. Crystal polymorphs of the compounds can be prepared by crystallization under different conditions.

Additionally, the compounds of the present invention, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

"Tautomers" refers to compounds whose structures differ markedly in arrangement of atoms, but which exist in easy and rapid equilibrium. It is to be understood that compounds of Formula I may be depicted as different tautomers. It should also be understood that when compounds have tautomeric forms, all tautomeric forms are intended to be within the scope of the invention, and the naming of the compounds does not exclude any tautomer form.

Some compounds of the present invention can exist in a tautomeric form which are also intended to be encompassed within the scope of the present invention.

The compounds and salts of the present invention can exist in several tautomeric forms, including the enol and imine form, and the keto and enamine form and geometric isomers and mixtures thereof. All such tautomeric forms are included within the scope of the present invention. Tautomers exist as mixtures of a tautomeric set in solution. In solid form, usually one tautomer predominates. Even though one tautomer may be described, the present invention includes all tautomers of the present compounds

A tautomer is one of two or more structural isomers that exist in equilibrium and are readily converted from one isomeric form to another. This reaction results in the formal migration of a hydrogen atom accompanied by a switch of adjacent conjugated double bonds. In solutions where tautomerization is possible, a chemical equilibrium of the tautomers will be reached. The exact ratio of the tautomers depends on several factors, including temperature, solvent, and pH. The concept of tautomers that are interconvertable by tautomerizations is called tautomerism.

Of the various types of tautomerism that are possible, two are commonly observed. In keto-enol tautomerism a simultaneous shift of electrons and a hydrogen atom occurs. Ring-chain tautomerism, is exhibited by glucose. It arises as a result of the aldehyde group (-CHO) in a sugar chain molecule reacting with one of the hydroxy groups (-OH) in the same molecule to give it a cyclic (ring-shaped) form.

Tautomerizations are catalyzed by: Base: 1. deprotonation; 2. formation of a delocalized anion (*e.g*. an enolate); 3. protonation at a different position of the anion; Acid: 1. protonation; 2. formation of a delocalized cation; 3. deprotonation at a different position adjacent to the cation.

Common tautomeric pairs are: ketone - enol, amide - nitrile, lactam - lactim, amide - imidic acid tautomerism in heterocyclic rings (*e.g*. in the nucleobases guanine, thymine, and cytosine), amine - enamine and enamine - enamine. Examples include:

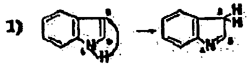

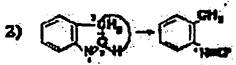

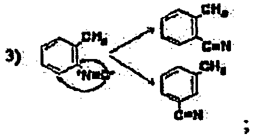

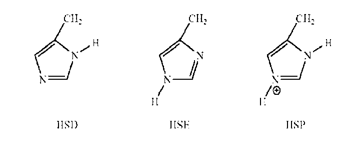

A "pharmaceutical composition" is a formulation containing the disclosed compounds in a form suitable for administration to a subject.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

The compounds of the invention are capable of further forming salts. All of these forms are also contemplated within the scope of the claimed invention. For example, the salt can be an acid addition salt. One example of an acid addition salt is a hydrochloride salt. Another example is a hydrobromide salt.

"Pharmaceutically acceptable salt" of a compound means a salt that is pharmaceutically acceptable and that possesses the desired pharmacological activity of the parent compound.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

Other examples include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The invention also encompasses salts formed when an acidic proton present in the parent compound either is replaced by a metal ion, *e.g*., an alkali metal ion, an alkaline earth ion, or an aluminum ion; or coordinates with an organic base such as ethanolamine, diethanolamine, triethanolamine, tromethamine, N-methylglucamine, and the like.

It should be understood that all references to pharmaceutically acceptable salts include solvent addition forms (solvates) or crystal forms (polymorphs) as defined herein, of the same salt.

The pharmaceutically acceptable salts of the present invention can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington's Pharmaceutical Sciences, 18th ed. (Mack Publishing Company, 1990). For example, salts can include, but are not limited to, the hydrochloride and acetate salts of the aliphatic amine-containing, hydroxyl amine-containing, and imine-containing compounds of the present invention.

Stable compound" and "stable structure" are meant to indicate a compound that is sufficiently robust to survive isolation to a useful degree of purity from a reaction mixture, and formulation into an efficacious therapeutic agent.

### Methods of Treating Cellulite

In addition to the currently investigated use of HP as a therapeutic for fibrosis-associated diseases, HF, febrifugine, and related compounds of formula (I) are effective agents in the prevention and treatment of the skin condition cellulite. It has been observed that HF prevents the spreading and contractile activity of dermal fibroblasts, a cellular function that is believed to mediate tissue changes during cellulite formation and progression. HF, febrifugine, and related compounds of formula (I) are used herein for the treatment of cellulite because 1) the action of HF and small molecules that are functionally related to HP, eg., molecules identified using the screening methods described herein, are antagonistic to the appearance of cellulite based on the model for cellulite formation and progression described above, 2) these small molecules are readily delivered to the cellulite-affected areas through a topical cream formulation and can be used in combination with treatments that improve the general appearance of the skin, i. e., laser and mechanical manipulation 3) these compounds can be used effectively and with low-toxicity for the treatment of cellulite whether they are delivered topically, orally, or through subcutaneous injection based on the human trial data for other applications of HF. The preferred method for the application of HF or related compounds of formula (I) is a topical cream containing either HF or its plant-derived precursor febrifugine. Oral therapy or subcutaneous injection would be alternative methods for the delivery of the cellulite antagonists.

Compositions formulated for treatment of cellulite are targeted for maximum penetrance in the hypodermis of the subject. For example, in topical compositions designed to treat the formation and/or progression of cellulite, the penetrance of each topical formulation is compared and tested for tissue bioavailability, e.g., by using pig skin, which is an excellent model for human skin. The penetrance of each formulation for the treatment and/or reduction of cellulite is tested, for example, using blood level analysis, animal models or a combination of both. For example, a formulation is applied to pig's skin, and after a suitable period post-application, a sample is removed from the test area, e.g., using a punch biopsy. The sample is then analyzed to detect the presence or absence of the formulation, and of the active ingredient in particular, within the skin sample. Alternatively or in addition to this biopsy procedure, blood samples are analyzed to determine whether the formulation, and in particular, the active ingredient of the formulation, e.g., HF or its plant-derived precursor febrifugine, is present in the blood, serum or other blood component of the subject. In human patients, penetrance of the formulation is determined, for example, using blood level analysis, biopsy analysis, or a combination of both.

Topical creams that prevent or reverse the characteristic dimpling seen with cellulite could be used in combination with treatments such as laser. The impact of laser treatments would be to act by increasing the amount of collagen in the dermis, helping to disguise the effects of the discontinuities at the dermo-hypodermal connective tissue border, a minimal protrusion of fat, by creating plumper skin. The ubiquitous gender-linked discontinuities of this connective tissue border likely will not be impacted by HF treatments, but these structural features are not, by themselves, responsible cellulite presentation. Laser treatments likely will be minimally effective alone, requiring frequent treatment for minor short-term improvement at greater expense. A combined treatment approach using laser treatment along with a mechanistically-based topical cream that directly addresses/reverses skin dimpling could improve the delivery and penetration of the active compound into the deep hypodermis, and provide a further general enhancement of the skin's appearance.

Without wishing to be bound by theory, laser treatment is thought to augment the penetration of a topically-applied ECM-remodeling compound of the invention. Further, topical creams or gels containing a compound of the invention capable of remodeling the ECM can be used in conjunction with laser treatments, mechanical manipulation, or both.

One tissue treatment involves the application of laser radiation to an area targeted for, cellulite reduction, in a predetermined wavelength, or combination of wavelengths, intervals, periods, and patterns. The application of infrared radiation can optionally follow. Optionally, subsequent to the application of infrared radiation, the tissue can be massaged using a combination of massage techniques.

The duration, power, and wavelengths of the application of the laser depends primarily on a formulation of different factors, such as calf circumference, thigh circumference, abdominal circumference, hip circumference, patient weight, and patient height.

For example, a patient having a thigh circumference of 38 cm, calf circumference of 31 cm, abdominal circumference of 76 cm, hip circumference of 82 cm, weight of 62 kg, ideal body weight of 50 kg according to Standard Metropolitan Life Insurance Company Tables, and height of 5 feet 3 inches, would be exposed to the rastered radiation for 6.4 minutes per area. The time duration for exposure of each area on the skin surface is calculated based on the following expression: (Hip Circumference in cm)/(Abdominal Circumference in cm) X (Weight in kgs)/(Ideal Body Weight in kgs) X (Calf Circumference in cm+Thigh Circumference in cm) X Reduction Factor=Time in Minutes. Ideal Body Weight ("IBW") is as defined by Standard Metropolitan Life Insurance Company tables for IBW calculated from the patient's height. The Reduction Factor is 0.07 with a minimum exposure of 5.5 minutes per area. The maximum exposure per area for a majority of the patients is about 12 minutes. However, this number may vary depending on the above variables. Again, the power of the laser 12 in the described embodiment is fixed. However, a laser having variable power settings can be employed, and the above relationship of variables adjusted for different power settings.

The typical exposure time per area being exposed is between about five and twelve minutes for an individual female adult of average build. For example, the thigh area can be treated in three sections, each area encompassing 120 degrees of the thigh with approximately 20% overlap. Thus, for each thigh, the laser treatment time is approximately three times the duration of exposure for each area, or eighteen to thirty minutes per limb. The duration of exposure varies depending on the actual values of the above-mentioned variables. The laser is delivered as a continuous wave beam rastered across the skin surface. The entire skin area to be treated may be covered by a single scan at a rate of about 1Hz.

Examples of laser systems useful in combination with the compounds of the invention include VELASMOOTH™ (Syneron Inc, Richmond Hill, Ontario, CA), which combines near infrared light at a wavelength of 700 nm, continuous wave radiofrequency and mechanical suction, and TRI-ACTIVE™ Laserdermology (Cynosure Inc., Chelmsford MA), which combines six near-infrared diode lasers at a wavelength of 810 nm, localized cooling, and mechanical massage.

### Methods of Treating or Preventing Stretch Marks

Topical delivery of HF, modified HF, febrifugine, or other functionally-related compounds in a vehicle with limited dermal penetrance are used to prevent and improve the appearance of stretch marks. In contrast to the condition cellulite, therapeutics for stretch marks need not gain access to tissues beneath the reticular dermis. In particular, compositions formulated for treatment of stretch marks are targeted for maximum penetrance in the dermis of the subject. Since the appearance of stretch marks occurs frequently and predictably during pregnancy, the design of any effective preventative, or therapeutic should take into account this physiologic condition. For protection of the fetus, limited tissue penetrance of a topical cosmetic agent is desirable for treatments intended for use during pregnancy, and can be obtained by modification of either the active compound or its application-vehicle.

The compositions disclosed herein include, for example, a limited-penetrance derivative of HF for the treatment of stretch marks. Alternatively or in addition, topical creams that contain febrifugine, HF, and HF derivatives are designed to have limited penetrance, e.g., the compound is preferentially retained at the dermis. The penetrance of each topical formulation is compared and tested for tissue bioavailability, for example, in pig skin, which is an excellent model for human skin. The penetrance of each topical formulation is compared and tested to ensure that the formulation is not present in the bloodstream of the subject or animal model. The penetrance of each formulation for the treatment of stretch marks is tested, for example, using blood level analysis, animal models or a combination of both. For example, a formulation designed to treat or prevent stretch marks is applied to pig's skin, and after a suitable period post-application, a sample is removed from the test area. The sample is then analyzed to determine to detect the presence or absence of the formulation, and of the active ingredient in particular, within the skin sample. Alternatively or in addition to this biopsy procedure, blood samples are analyzed to determine whether the formulation, and in particular, the active ingredient of the formulation, e.g., HF or its plant-derived precursor febrifugine, is present in the blood, serum or other blood component of the subject. In human patients, penetrance of the formulation for treating or preventing stretch marks is determined, for example, using blood level analysis.

The methods for preventing the appearance of or reducing the appearance of stretch marks can be used in conjunction with a second treatment regime. For example, the compositions of provided herein can be used in conjunction with dermabrasion, chemical peel, and/or laser treatments. The methods for preventing the appearance of or reducing the appearance of stretch marks can be used in conjunction with other topical agents such as, e.g., cocoa butter, compositions comprising vitamin A and/or vitamin E, and other commercially available over the counter products.

### Determination of the Biological Effect of an Anti-Cellulite Therapeutic

In various embodiments of the invention, suitable *in vitro* studies are performed to determine the effect of a specific antl-cellulite therapeutic and whether its administration is indicated for treatment of the affected tissue exhibiting cellulite. For example, the biological effect of an anti-cellulite therapeutic, such as HF, is monitored by measuring the contractile force exerted by vertical collagen fibers tethered between fascia and dermo-hypodermal interface in a tissue site that has been treated with the therapeutic. The biological effect of a therapeutic is also measured by observing the tissue structure after treatment using, for example, magnetic resonance imaging (MRI) of the treated area. Alternatively, or in addition, the biological effect is monitored by physical observation of the appearance of the treated tissue site *e.g*., observing the extent of skin dimpling within the treated area and/or observing the depth of each dimple in the treated area. Typically, the observation is performed by a plastic surgeon.

### Pharmaceutical Compositions and Formulations

The modulators of cellulite formation and/or progression and modulator of stretch mark formation and/or progression can be incorporated into pharmaceutical compositions suitable for administration. Such compositions typically comprise the modulator and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences. Preferred examples of such carriers or diluents include, but are not limited to, water, saline, finger's solutions, dextrose solution, and 5% human serum albumin. Liposomes and non aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions. Pharmaceutical compositions containing one or more active ingredients, *e.g*., one or more modulators of cellulite formation and/or progression, are formulated as prescription formulations, or alternatively as over-the-counter formulations.

A pharmaceutical composition formulated to be compatible with its intended route of administration. Examples of routes of administration include parenteral, *e.g*., intravenous, intradernnal, subcutaneous, oral (*e.g*., inhalation), transdermal (*i.e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Formulations suitable for topical administration include liquid or semi-liquid preparations such as liniments, lotions, gels, applicants, oil-in-water or water-in-oil emulsions such as creams, ointments or pastes; or solutions or suspensions such as drops. Formulations for topical administration to the skin surface can be prepared by dispersing the drug with a dermatologically acceptable carrier such as a lotion, cream, ointment or soap. Useful are carriers capable of forming a film or layer over the skin to localize application and inhibit removal. For topical administration to internal tissue surfaces, the agent can be dispersed in a liquid tissue adhesive or other substance known to enhance adsorption to a tissue surface. For example, hydroxypropylcellulose or fibrinogen/thrombin solutions can be used to advantage. Alternatively, tissue-coating solutions, such as pectin-containing formulations can be used.

Additionally, the carrier for a topical formulation can be in the form of a hydroalcoholic system (*e.g*. quids and gels), an anhydrous oil or silicone based system, or an emulsion system, including, but not limited to, oil-in-water, water-in-oil, water-in-oil-in-water, and oil-in-water-in-silicone emulsions. The emulsions can cover a broad range of consistencies including thin lotions (which can also be suitable for spray or aerosol delivery), creamy lotions, light creams, heavy creams, and the like. The emulsions can also include microemulsion systems. Other suitable topical carriers include anhydrous solids and semisolids (such as gels and sticks); and aqueous based mousse systems. Nonlimiting examples of the topical carrier systems useful in the present invention are described in the following four references: "Sun Products Formulary", Cosmetics & Toiletries, vol. 105, pp. 122-139 (December 1990); "Sun Products Formulary", Cosmetics & Toiletries, vol. 102, pp. 117-136 (March 1987); U.S. Pat. No. 4,960,764; and U.S. Pat. No. 4,254,105.

The following components are useful for topical compositions:

### Humectants, moisturizers, and skin conditioners

Particularly for topical compositions, optional component of the compositions useful in the instant invention is at least one humectant/moisturizer/skin conditioner. A variety of these materials can be employed and each can be present at a level of from about 0.1% to about 20%, alternatively from about 1% to about 10% and yet alternatively from about 2% to about 5%. These materials include urea; guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); polyhydroxy alcohols such as sorbitol, glycerol, hexanetriol, propylene glycol, hexylene glycol and the like; polyethylene glycol; sugars and starches; sugar and starch derivatives (*e.g.*, alkoxylated glucose); hyaluronic acid; lactamide monoethanolamine; acetamide monoethanolamine; and mixtures thereof.

In certain topical compositions, humectants/moisturizers/skin conditioners useful herein are the C₃ -C₆ diols and triols, and also aloe vera gel. Especially preferred is the triol, glycerol, and also aloe vera gel.

### Surfactants

The compositions useful in the methods of the present invention, particularly the topical compositions, can optionally comprise one or more surfactants. The surfactants can be present at a level from about 0.1% to about 10%, alternatively from about 0.2% to about 5%, and yet alternatively from about 0.2% to about 2.5%. Suitable surfactants include, but are not limited to, nonionic surfactants such as polyalkylene glycol others of fatty alcohols, and anionic surfactants such as taurates and alkyl sulfates. Nonlimiting examples of these surfactants include isoceteth-20, sodium methyl cocoyl taurate, sodium methyl oleoyl taurate, and sodium lauryl sulfate. *See* U.S. Pat. No. 4,800,197. Examples of a broad variety of additional surfactants useful herein are described in McCutcheon's, Detergents and Emulsifiers, North American Edition (1986), published by Allured Publishing Corporation..

### Emollients

The compositions useful in the methods of the present invention, particularly topical compositions, can also optionally comprise at least one emollient. Examples of suitable emollients include, but are not limited to, volatile and non-volatile silicone oils, highly branched hydrocarbons, and non-polar carboxylic acid and alcohol esters, and mixtures thereof. Emollients useful in the instant invention are further described in U.S. Pat. No. 4,919,934.

The emollients can typically comprise in total from about 1% to about 50%, preferably from about 1% to about 25%, and more preferably from about 1% to about 10% by weight of the compositions useful in the present invention.

### Sunscreens

The compositions useful in the methods of the present invention for topical administration can also optionally comprise at least one sun screening agent. A wide variety of one or more sun screening agents are suitable for use in the present invention and are described in U.S. Pat. No. 5,087,445; U.S. Pat. No. 5,073,372; U.S. Pat. No. 5,073,371; and Segarin, et al., at Chapter VIII, pages 189 et seq., of Cosmetics Science and Technology.

Certain useful in the compositions of the instant invention ethylhexyl p-methoxycinnamate, octocrylene, octyl salicylate, oxybenzone, or mixtures thereof. Other useful sunscreens include the solid physical sunblocks such as titanium dioxide (micronized titanium dioxide, 0.03 microns), zinc oxide, silica, iron oxide and the like. Without being limited by theory, it is believed that these inorganic materials provide a sun screening benefit through reflecting, scattering, and absorbing harmful UV, visible, and infrared radiation.

Still other useful sunscreens are those disclosed in U.S. Pat. No. 4,937,370; and U.S. Pat. No. 4,999,186. The sun screening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range. These sun screening agents provide higher efficacy, broader UV absorption, lower skin penetration and longer lasting efficacy relative to conventional sunscreens.

Generally, the sunscreens can comprise from about 0.5% to about 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, Aug. 25, 1978.

The topical compositions useful for the methods of the instant invention can also be delivered from a variety of delivery devices. For example, the compositions useful herein can be incorporated into a medicated cleansing pad. Preferably these pads comprise from about 50% to about 75% by weight of one or more layers of nonwoven fabric material and from about 20% to about 75% by weight (on dry solids basis) of a water soluble polymeric resin. These pads are described in detail in U.S. Pat. No. 4,891,228 and U.S. Pat. No. 4,891,227. The compositions useful herein can also be incorporated into and delivered from a soft-tipped or flexible dispensing device. These devices are useful for the controlled delivery of the compositions to the skin surface and have the advantage that the treatment composition itself never need be directly handled by the user. Nonlimiting examples of these devices comprise a fluid container including a mouth, an applicator, means for holding the applicator in the mouth of the container, and a normally closed pressure-responsive valve for permitting the flow of fluid from the container to the applicator upon the application of pressure to the valve. The valve can include a diaphragm formed from an elastically fluid impermeable material with a plurality of non-intersecting arcuate slits therein, where each slit has a base which is intersected by at least one other slit, and where each slit is out of intersecting relation with its own base, and wherein there is a means for disposing the valve in the container inside of the applicator. Examples of these applicator devices are described in U.S. Pat. No. 4,693,623; U.S. Pat. No. 4,620,648; U.S. Pat No. 3,669,323; U.S. Pat. No. 3,418,055; and U.S. Pat. No. 3,410,645. Examples of applicators useful herein are commercially available from Dab-O-Matic, Mount Vernon, N.Y.

For example, halofuginone is formulated for topical administration as a cream containing 0.03% halofuginone in a paraffin/water base daily for 60 days. (*See e.g.,* Nagler and Pines, Transplantation, vol. 68(11):1806-9 (1999)). In the formulations for topical administration, halofuginone is present in an amount between 0.01% and 100% of the total composition. For example, the dosage of halofuginone is in the range of 0.01% to 50 %; 0.01% to 25%; 0.01% to 10%; 0.01% to 5%; 0.01% to 2%; 0.01% to 1.5%; 0.01% to 1%; 0.01% to 0.75%; 0.01% to 0.5%; 0.01% to 0.25%; 0.01% to 0.1%; 0.01 % to 0.09%; 0.01 % to 0.0 8%; 0.01% to 0.07%; 0. 01 % to 0. 06%; 0.01% to 0.05%; 0.01 % to 0.04%; 0.01 % to 0.03%; and 0.01 % to 0.02%. As described above, the effect of the halofuginone cream is evaluated by photography/visual inspection by a plastic surgeon.

The invention is further described in the following examples, which do not limit the scope of the invention described in the claims.

### EXAMPLES

### Example 1. The Effect of HF on fibroblast behavior

*Assay of HF effects on contractile behavior of dermal fibroblasts*. Previous studies have established that HF can modulate fibrosis *in vivo*, and regulate ECM production by cells *in vitro.* The studies described herein provide a simple assay to test the effects of HF and its putative mediators on the behavior of cells likely to provide the basis for the structural features of cellulite- dermal fibroblasts and myofibroblasts. When human primary dermal fibroblasts are cultured in a matrix of Type I collagen, mimicking the interstitial matrix of the dermis and hypodermis, they attach to the substratum and spread, exerting contractile force on the surrounding interstitial matrix (Fig. 2A). Primary dermal fibroblasts were incubated on a matrix of Type I collagen for 24 hrs in the presence of a 200 nM MAZ1310 (an inactive derivative of halofuginone) (Panel A), or 200 nM halofuginone (Panel B). When cultured in the presence of 200 nM HF, however, dermal fibroblasts completely fail to attach and spread, instead they remain completely spherical (Fig. 2B). Thus, halofuginone completely prevented the ability of the fibroblasts to spread on the collagen matrix. Identical results were seen when using as little as 30 nM halofuginone.

Fibroblasts differentiate into myofibroblasts in response to a stimulus of cytokine release, mechanical stress, or a combination of the two triggers. By preventing fibroblasts from attaching to the surrounding matrix, HF thereby prevents the transmission of mechanical stress in the matrix to the fibroblasts, preventing fibroblast differentiation to myofibroblasts. This assay, therefore, provides a convenient, direct approach to study how HF-regulated pathways inhibit myofibroblast maturation/activity, prevent or reverse the dynamic changes in hypodermal structure that form the basis for skin dimpling known as cellulite.

### Example 2. Identification of candidate mediators of HF action

The molecular mechanisms underlying the regulation of ECM by HF currently are not known. To make possible the design of more effective or alternative approaches to small molecule inhibition of ECM production and contractile activity of fibroblasts, the first HF binding proteins have been identified herein. These proteins, TIF1ß and Ruvb12, are both transcriptional regulatory proteins. TIF1ß is known to interact with transcription factors that regulate Type I collagen expression, providing the first molecular connection between HF and the regulation of an ECM gene. A brief description and data detailing the isolation and identification of HF binding proteins are provided below. Using an affinity purification approach, the studies described herein have identified protein bands that bind to an HF affinity resin, do not bind to control resin, and are specifically competed for binding to HF affinity resin by excess free HF (Fig. 3). Primary dermal SV-NIES cell lysates were bound to HF-affinity resin or control resin in the presence or absence of free HF or the inactive derivative HFd4. The arrow in Figure 3 depicts a band that binds only to HF-resin and in competed by active, but not inactive HF. This band was identified by tandem mass spectrometry as TIF1β.

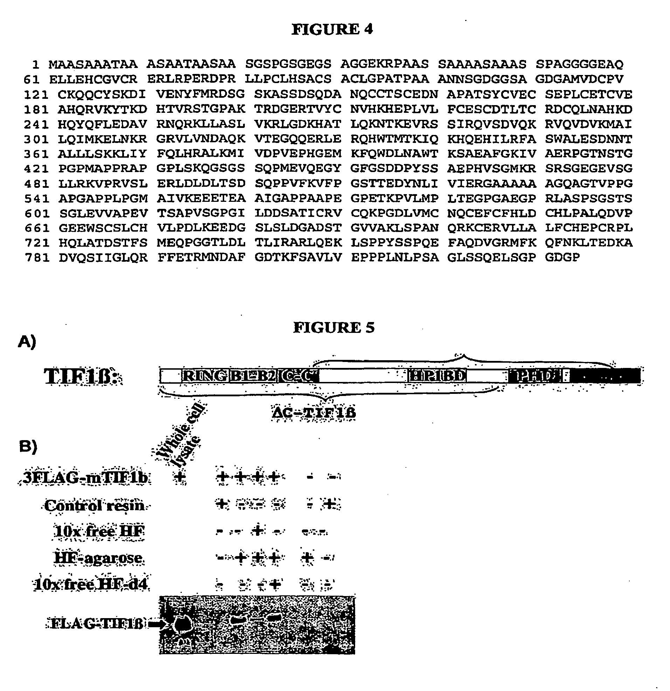

These HF binding proteins were isolated and identified by tandem mass spectrometry. Using this approach, the transcriptional co-repressor TIF1ß (Fig. 4) was initially identified as an HF binding protein. A cDNA encoding TIF1ß was also identified and used to confirm that TIF1ß binds specifically to HF (Fig. 5). Cloned, tagged TIF1β was transfected into SV-MES cells and precipitated with HF-resin. No binding was seen with control resin, and pre-incubation with HF, but not the inactive derivative HF-d4, blocked TIF1β interaction with immobilized HF.

TIF1ß is a member of a family of transcriptional co-repressors (Chang, et al., Mol Cell Biol, vol. 18: 5880-87 (1998); Hsu, et al. Embo J., vol. 20: 2273-85 (2001); Le Douarin, et al. J Steroid Biochem Mol Biol 65,43-50(1998)). TIT1ß interacts with a variety of transcription factors, including nuclear receptors, to mediate regulation of chromatin modification (Chang, et al., Mol Cell Biol, vol. 18: 5880-87 (1998); Hsu, et al. Embo J., vol. 20: 2273-85 (2001); Nomura, et al. J Biol Chem 279, 16715-26 (2004)). TIF1ß interacts with a number of transcription factors known to regulate the synthesis of Type I collagen, providing a direct link between HF, TIF1ß and the transcription of extracellular matrix genes. The affinity purification/tandem mass spectrometry approach was also used to identify a second HF binding protein as RuvB12. Ruvb12, also known as Reptin, ECP51, Tip48, and Tip49b, is, like TIP1ß, known to function in transcriptional regulation and chromatin re-modeling (Kanemaki, et al. J Biol Chem 274, 22437-44 (1999)). The identification of halofuginone's intracellular effectors, and the binding sites for halofuginone in those effectors, provides additional approaches to targeting the deposition of sub-dermal ECM. New classes of small molecules can be designed to target intracellular effectors such as TIF1ß and RuvB12, broadening the range of potential therapeutic agents for the treatment of cellulite.

### Example 3. The effect of HF on myofibroblasts formation in response to TGFβ

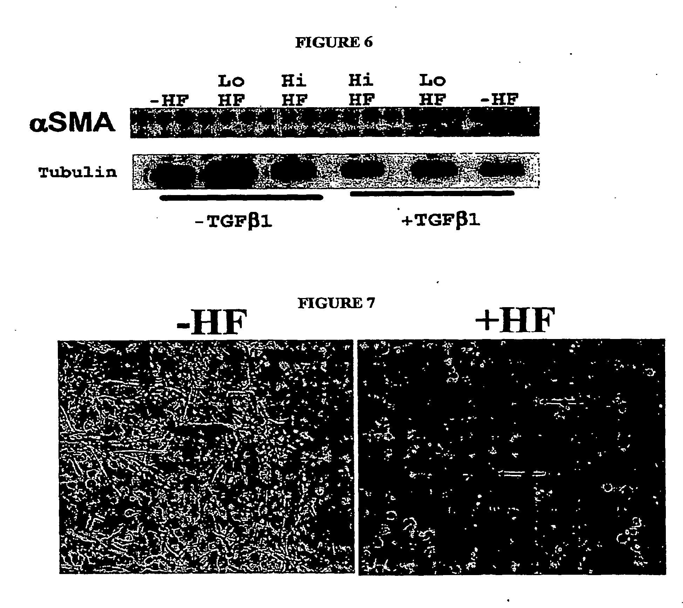

NMuMg murine epithelial cells were induced to undergo transition to myofibroblasts by stimulation with 5 ng/ml TGFß1. After 72 hours, myofibroblast differentiation was assessed by Western blot for Smooth muscle actin (SMA), a myofibroblast contractile protein. As seen in Figure 6, increasing doses of HF eliminated SMA induction. Thus, HF inhibited myofibroblasts formation in response to TGFβ.

### Example 4. The effect of HF on TGFβ-treated dermal fibroblasts

Primary human dermal fibroblasts were seeded in a matrix of Type I collagen with 5 ng/ml TGFß1 in the presence or absence of 100 nM HF. Figure 7 depicts the effect of the presence and absence of HF on the behavior of TGFβ-treated dermal fibroblasts. In the absence of HF, bundles of extended contractile fibroblasts were observed (arrow in left panel of Fig. 7); in the presence of HF fibroblasts were fully viable, but showed no extensions or attachments to the matrix or to one another (arrows in right panel of Fig. 7).

### Example 5. The effect of HF on contractile activity of myofibroblasts

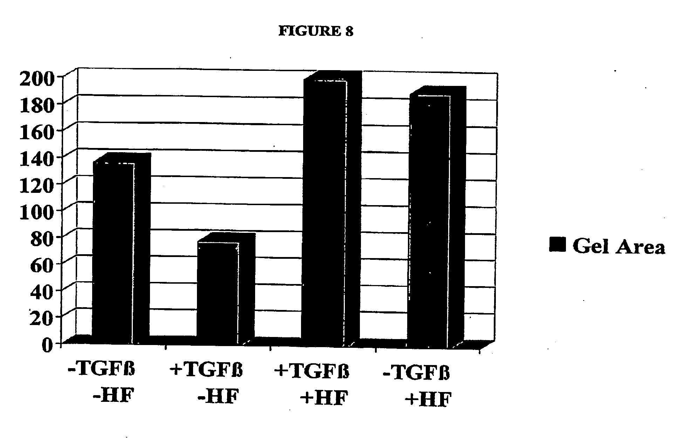

Human primary dermal fibroblasts were seeded in a matrix of Type I collagen in the presence or absence of 5 ng/ml TGFß or 100 nM HF. Collagen discs were released from attachment to the culture dish and allowed to undergo fibroblast-driven contraction for 72 hours. Disc diameter was measured, and the area of the disc was calculated and presented in arbitrary units shown in Figure 8. The area of the discs at time zero was 200 units. Thus, HF inhibited the contractile activity of myofibroblasts in a collagen matrix.

## Claims

1. A non-therapeutic method for decreasing the appearance of cellulite in a subject exhibiting cellulite, said method comprising:
(a) identifying a tissue treatment site in said patient, wherein said tissue treatment site comprises at least one area exhibiting cellulite;
(b) contacting the tissue treatment site with a compound of formula I, 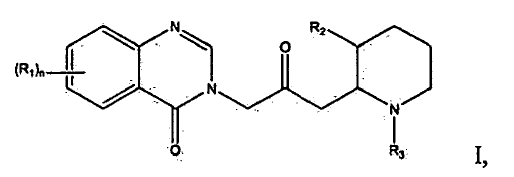 or a salt, isomer or solvate thereof,
wherein: R₁ is selected from hydrogen, halogen, nitro, benzo, C₁₋₆ alkyl, phenyl and C₁₋₆ alkoxy;
R₂ is selected from hydroxy, acetoxy, and C₁₋₆ alkoxy,
R₃ is hydrogen, and
n is selected from 1, 2, 3 and 4.
wherein the treated tissue site exhibits a decreased contractile force exerted by vertical collagen fibers tethered between fascia and dermo-hypodermal interface within the tissue treatment site, as compared to a contractile force exerted by vertical collagen fibers in a second tissue site exhibiting cellulite in said subject.

2. A non-therapeutic method for preventing or decreasing the appearance of a stretch mark in a subject comprising administering a compound that modulates the formation or progression of stretch marks, wherein said compound is a compound of formula I: 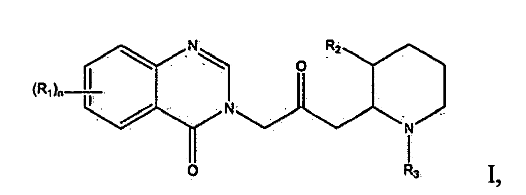 or a salt, isomer or solvate thereof,
wherein: R₁ is selected from hydrogen, halogen, nitro, benzo, C₁₋₆ alkyl, phenyl and C₁₋₆ alkoxy;
R₂ is selected from hydroxy, acetoxy, and C₁₋₆ alkoxy,
R₃ is hydrogen, and
n is selected from 1, 2, 3 and 4;
in an amount effective to modulate the formation and/or progression of cellulite in a subject.

3. The method of claim 1 or claim 2, wherein said compound is febrifugine, or said compound is halofuginone.

4. The method of claim 1 or claim 2, wherein said compound inhibits maturation of myofibroblasts.

5. The method of claim 1 or claim 2, wherein said compound inhibits one or more biological activities of myofibroblasts.

6. The method of claim 5, wherein said biological activity is selected from expression of actin-containing stress fibers, expression and organization of fibronectin into fibrils, and formation of large fibronexus adhesion complexes.

7. The method of claim 1 or claim 2, wherein said compound is formulated as a film, membrane, foam, gel, or cream.

8. The method of claim 1, further comprising the step of:
(c) exposing said tissue treatment site to a laser.

9. The method of claim 1, further comprising the step of:
(c) exposing said tissue treatment site to mechanical manipulation.

## Patentansprüche

1. Nicht-therapeutisches Verfahren zur Verminderung des Erscheinungsbilds von Cellulite bei einem Individuum, das Cellulite hat, wobei das Verfahren aufweist:
(a) Identifizieren einer Gewebe-Behandlungsstelle bei dem Patienten, wobei die Gewebe-Behandlungsstelle mindestens einen Bereich aufweist, der Cellulite zeigt;
(b) in Berührung bringen der Gewebe-Behandlungsstelle mit einer Verbindung der Formel I 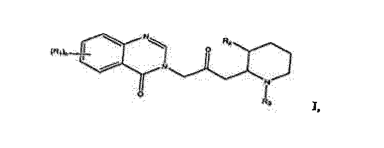 oder einem Salz, Isomer oder Solvat davon,
wobei:
R₁ ausgewählt wird aus Wasserstoff, Halogen, Nitro, Benzo, C₁₋₆-Alkyl, Phenyl und C₁₋₆-Alkoxy;
R₂ ausgewählt wird aus Hydroxy, Acetoxy und C₁₋₆-Alkoxy,
R₃ Wasserstoff ist, und
n ausgewählt wird aus 1, 2, 3 und 4,
wobei die behandelte Gewebestelle eine verringerte Kontraktionskraft zeigt, die von vertikalen Kollagenfasern ausgeübt wird, die an der Gewebe-Behandlungsstelle zwischen Faszie und dermato-hypodermaler Grenzfläche gebunden sind, im Vergleich zu einer Kontraktionskraft, die von vertikalen Kollagenfasern an einer zweiten Gewebestelle bei dem Individuum, die Cellulite zeigt, ausgeübt wird.

2. Nicht-therapeutisches Verfahren zur Verhinderung oder Verminderung des Erscheinungsbilds eines Dehnungsstreifens bei einem Individuum, aufweisend ein Verabreichen einer Verbindung, die die Bildung oder Weiterentwicklung von Dehnungsstreifen reguliert, wobei die Verbindung eine Verbindung der Formel I ist: 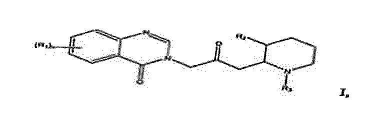 oder ein Salz, Isomer oder Solvat davon,
wobei:
R₁ ausgewählt wird aus Wasserstoff, Halogen, Nitro, Benzo, C₁₋₆-Alkyl, Phenyl und C₁₋₆-Alkoxy;
R₂ ausgewählt wird aus Hydroxy, Acetoxy und C₁₋₆-Alkoxy,
R₃ Wasserstoff ist, und
n ausgewählt wird aus 1, 2, 3 und 4;
in einer Menge, die wirksam ist, die Bildung und/oder Weiterentwicklung von Cellulite bei einem Individuum zu regulieren.

3. Verfahren nach Anspruch 1 oder Anspruch 2, bei dem die Verbindung Febrifugin ist oder die Verbindung Halofuginon ist.

4. Verfahren nach Anspruch 1 oder Anspruch 2, bei dem die Verbindung die Reifung von Myofibroblasten hemmt.

5. Verfahren nach Anspruch 1 oder Anspruch 2, bei dem die Verbindung eine oder mehrere biologische Wirkungen von Myofibroblasten hemmt.

6. Verfahren nach Anspruch 5, bei dem die biologische Wirkung ausgewählt wird aus der Expression von Actin enthaltenden Streßfasern, der Expression und Anordnung von Fibronectin zu Fibrillen, und der Bildung großer Fibronexus-Adhäsionskomplexe.

7. Verfahren nach Anspruch 1 oder Anspruch 2, bei dem die Verbindung als ein Film, eine Membran, ein Schaum, ein Gel oder eine Creme formuliert wird.

8. Verfahren nach Anspruch 1, außerdem folgenden Schritt aufweisend:
(c) Laser-Exposition der Gewebe-Behandlungsstelle.

9. Verfahren nach Anspruch 1, außerdem folgenden Schritt aufweisend:
(c) Exponieren der Gewebe-Behandlungsstelle einer mechanischen Manipulation.

## Revendications

1. Procédé non thérapeutique pour réduire l'aspect de cellulite chez un sujet présentant de la cellulite, ledit procédé comprenant :
(a) l'identification d'un site de traitement tissulaire chez ledit patient, ledit site de traitement tissulaire comprenant au moins une zone présentant de la cellulite ;
(b) la mise en contact du site de traitement tissulaire avec un composé de formule I: 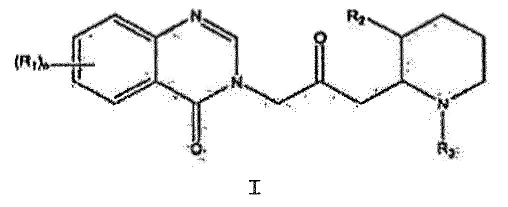 ou un sel, isomère ou solvate de celui-ci,
formule dans laquelle :
R₁ est choisi parmi l'hydrogène, un halogène, nitro, benzo, alkyle en C₁ à C₆, phényle et alcoxy en C₁ à C₆ ;
R₂ est choisi parmi hydroxy, acétoxy, et alcoxy en C₁ à C₆, R₃ est l'hydrogène, et
n est choisi parmi 1, 2, 3 et 4,
dans lequel le site tissulaire traité présente une diminution de la force contractile exercée par des fibres de collagène verticales attachées entre le fascia et l'interface dermo-hypodermique à l'intérieur du site de traitement tissulaire, en comparaison avec la force contractile exercée par des fibres de collagène verticales dans un deuxième site tissulaire présentant de la cellulite chez ledit sujet.

2. Procédé non thérapeutique pour prévenir ou réduire l'apparition d'une vergeture chez un sujet, comprenant l'administration d'un composé qui module la formation ou la progression des vergetures, dans lequel ledit composé est un composé de formule I : 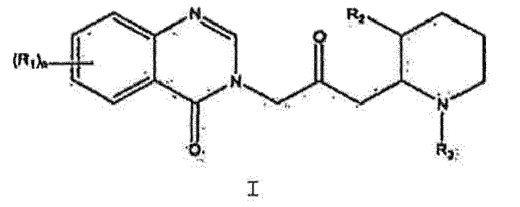 ou un sel, isomère ou solvate de celui-ci,
formule dans laquelle :
R₁ est choisi parmi l'hydrogène, un halogène, nitro, benzo, alkyle en C₁ à C₆, phényle et alcoxy en C₁ à C₆ ;
R₂ est choisi parmi hydroxy, acétoxy, et alcoxy en C₁ à C₆,
R₃ est l'hydrogène, et
n est choisi parmi 1, 2, 3 et 4,
en une quantité efficace pour moduler la formation et/ou la progression de la cellulite chez un sujet.

3. Procédé selon la revendication 1 ou la revendication 2, dans lequel ledit composé est la fébrifugine, ou ledit composé est l'halofuginone.

4. Procédé selon la revendication 1 ou la revendication 2, dans lequel ledit composé inhibe la maturation des myofibroblastes.

5. Procédé selon la revendication 1 ou la revendication 2, dans lequel ledit composé inhibe une ou plusieurs activités biologiques des myofibroblastes.

6. Procédé selon la revendication 5, dans lequel ladite activité biologique est choisie parmi l'expression de fibres de stress contenant de l'actine, l'expression et l'organisation de fibronectine en fibrilles, et la formation de gros complexes d'adhésion de type fibronexus.

7. Procédé selon la revendication 1 ou la revendication 2, dans lequel ledit composé formulé sous la forme d'un film, d'une membrane, d'une mousse, d'un gel ou d'une crème.

8. Procédé selon la revendication 1, comprenant en outre l'étape consistant à :
(c) exposer ledit site de traitement tissulaire à un laser.

9. Procédé selon la revendication 1, comprenant en outre l'étape consistant à :
(c) exposer ledit site de traitement tissulaire à une manipulation mécanique.
